# EUROPÄISCHE PATENTANMELDUNG

(11) **EP 4 393 933 A1**
(43) Veröffentlichungstag der Anmeldung: **03.07.2024**
(21) Anmeldenummer: 22216770.2
(22) Anmeldetag: 27.12.2022
(51) Int. Cl.: C07F 9/6574, B01J 31/18, C07B 41/06, C07C 45/50, C07C 47/02

(54) **BISPHOSPHITE MIT DIOXAPHOSPHINAN-REST**

(71) Anmelder: Evonik Operations GmbH, 45128 Essen (DE)
(72) Erfinder: SALE, Anna Chiara, 45657 Recklinghausen (DE); FRANKE, Robert, 45772 Marl (DE); FRIDAG, Dirk, 45721 Haltern am See (DE); KUCMIERCZYK, Peter, 44628 Herne (DE); MARKOVIC, Ana, 45721 Haltern am See (DE); BÖRNER, Armin, 18059 Rostock (DE); SELENT, Detlef, 18059 Rostock (DE); ROMEIKE, Kerstin, 18109 Rostock (DE); WENZEL, Gudrun, 18196 Kessin (DE)
(74) Vertreter: Evonik Patent Association

(57) **Zusammenfassung**

Bisphosphite mit Dioxaphosphinan-Rest und deren Einsatz in der Hydroformylierung.

## Beschreibung

Die Erfindung betrifft Bisphosphite mit Dioxaphosphinan-Rest und deren Einsatz in der Hydroformylierung.

Phosphorhaltige Verbindungen spielen als Liganden in einer Vielzahl von Reaktionen eine entscheidende Rolle, z.B. in der Hydrierung, in der Hydrocyanierung und auch in der Hydroformylierung.

Die Reaktionen zwischen Olefinverbindungen, Kohlenmonoxid und Wasserstoff in Gegenwart eines Katalysators zu den um ein C-Atom reicheren Aldehyden ist als Hydroformylierung bzw. Oxierung bekannt. Als Katalysatoren in diesen Reaktionen werden häufig Verbindungen der Übergangsmetalle der VIII. Gruppe des Periodensystems der Elemente verwendet. Bekannte Liganden sind beispielsweise Verbindungen aus den Klassen der Phosphine, Phosphite und Phosphonite. Eine gute Übersicht über den Stand der Hydroformylierung von Olefinen findet sich in R. Franke, D. Selent, A. Börner, "Applied Hydroformylation", Chem. Rev., 2012 (112), 11, 5675-5732, DOI:10.1021/cr3001803.

Die technische Aufgabe der Erfindung ist die Bereitstellung einer Verbindung, mit welcher bei der Hydroformylierung von Olefinen eine gesteigerte Ausbeute an Aldehyd erzielt werden kann.

Die Aufgabe wird gelöst durch eine Verbindung gemäß Anspruch 1.

Verbindung gemäß Formel (**I**): 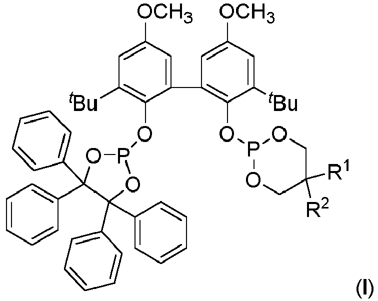 wobei R¹ für einen Phenylrest oder Naphthylrest steht, und R² für -H steht.

In einer Ausführungsform weist die Verbindung die Struktur (1) auf:

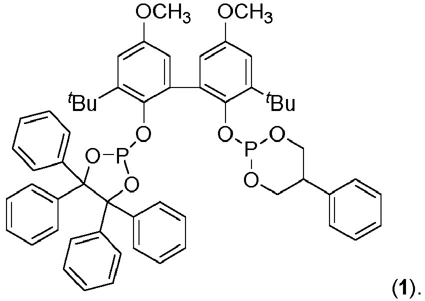

Neben der Verbindung selbst, wird auch ein Verfahren beansprucht, in welchem die zuvor beschriebenen Verbindungen zum Einsatz kommen.

Verfahren umfassend die Verfahrensschritte:
a) Vorlegen eines Olefins;
b) Zugabe einer zuvor beschriebenen Verbindung;
c) Zugabe einer Rh-Verbindung;
d) Zuführen von H₂ und CO;
e) Erwärmen des Reaktionsgemisches aus a) bis d), wobei das Olefin zu einem Aldehyd umgesetzt wird.

In einer Variante des Verfahrens ist die Rh-Verbindung ausgewählt aus: Rh(acac)(CO)₂, [(acac)Rh(COD)] (Umicore, acac = Acetylacetonat-Anion; COD = 1,5-Cyclooctadien), Rh₄CO₁₂.

In einer Variante des Verfahrens ist die Rh-Verbindung Rh(acac)(COD).

In einer Variante des Verfahrens erfolgt das Zuführen von H₂ und CO in Verfahrensschritt d) mit einem Druck in dem Bereich von 1 bis 6 MPa (10 bis 60 bar).

In einer Variante des Verfahrens erfolgt das Zuführen von H₂ und CO in Verfahrensschritt d) mit einem Druck in dem Bereich von 1,5 bis 4,5 MPa (15 bis 45 bar).

In einer Variante des Verfahrens erfolgt das Erwärmen des Reaktionsgemisches in Verfahrensschritt e) auf eine Temperatur in dem Bereich von 80 °C bis 160 °C.

In einer Variante des Verfahrens erfolgt das Erwärmen des Reaktionsgemisches in Verfahrensschritt e) auf eine Temperatur in dem Bereich von 100 °C bis 140 °C.

Im Folgenden soll die Erfindung anhand von Ausführungsbeispielen näher erläutert werden.

### Synthese Liganden

### Ligand (1): 2-((3,3'-Di-tert-butyl-5,5'-dimethoxy-2'-((4,4,5,5-tetraphenyl-1,3,2-dioxaphospholan-2-yl)oxy)-[1,1'-biphenyl]-2-yl)oxy)-5-phenyl-1,3,2-dioxaphosphinan

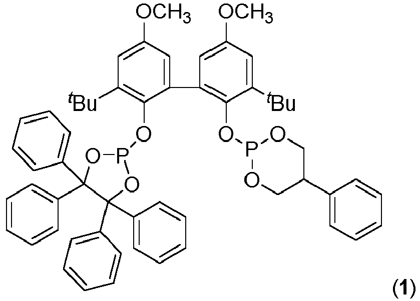

Zu einer Lösung von 4,4,5,5-Tetraphenyl-2-((3,3',5,5'-tetra-tert-butyl-2'-((dichlorophosphaneyl)oxy)-[1,1'-biphenyl]-2-yl)oxy)-1,3,2-dioxaphospholan (0,4361 g; 0,5108 mmol) in 5 ml Toluol wird bei Raumtemperatur tropfenweise eine Mischung von 2-Phenylpropandiol-1,2 (0,0777 g; 0,5108 mmol) und Triethylamin (0,28 ml) in 2 ml Toluol gegeben. Man rührt über Nacht, filtriert und engt das Filtrat im Vakuum zur Trockne ein. Der erhaltene Feststoff wird 2 h bei 60°C/0,1mbar getrocknet anschließend 1 h mit 4 ml Heptan verrührt. Ausbeute: 0,37 g (0,396 mmol; 77%).

ESI-TOF HRMS: m/z=933,3684; [M⁺+H], berechnet m/z=933,3679 und m/z=955,3489; [M⁺+Na], berechnet m/z=955,3499.

³¹P-NMR (CD₂Cl₂): 116,3 (d, *J*_{PP}=17 Hz); 123,0 (d, *J*_{PP}=19 Hz); 145,3 (d, *J*_{PP}=17 Hz); 145,3 (d, *J*_{PP}=19Hz) ppm.

¹H-NMR (CD₂Cl₂): 1,07+1,12 (2s, 9H); 1,33+1,37 (2s, 9H); 3,54+3,57 (2s, 6H); 2,94-4,60 (m, 5H); 3,60+3,64 (2s, 6H); 6,58 (m, 2H); 6,78-7,33 (m, 27H) ppm.

### Ligand (2): 2-((3,3'-Di-tert-butyl-5,5'-dimethoxy-2'-((4,4,5,5-tetraphenyl-1,3,2-dioxaphospholan-2-yl)oxy)-[1,1'-biphenyl]-2-yl)oxy)-5,5-dimethyl-1,3,2-dioxaphosphinan

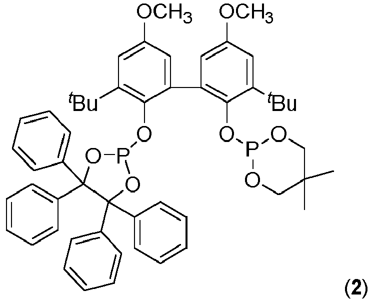

Zu einer Lösung von 4,4,5,5-Tetraphenyl-2-((3,3',5,5'-tetra-tert-butyl-2'-((dichlorophosphaneyl)oxy)-[1,1'-biphenyl]-2-yl)oxy)-1,3,2-dioxaphospholan (0,445 g; 0,5212 mmol) in 5 ml Toluol wird bei Raumtemperatur tropfenweise eine Mischung von 2,2'-Dimethylpropandiol-1,2 (0,0543 g; 0,5212 mmol) und Triethylamin (0,29 ml) in 2 ml Toluol gegeben. Man rührt über Nacht, filtriert und engt das Filtrat im Vakuum zur Trockne ein. Der erhaltene Feststoff wird 2 h bei 60°C/ 0,1 mbar getrocknet. Ausbeute: 0,410 g (0,463 mmol, 89%).

ESI-TOF HRMS: m/z=907,3514; [M⁺+Na], berechnet m/z=907,3499.

³¹P-NMR (CD₂Cl₂): 116,6 (d, *J*_{PP}=19 Hz); 145,6 (d, *J*_{PP}=19 Hz) ppm.

¹H-NMR (CD₂Cl₂): 0,75 (s; 3H); 1,20 (s; 3H); 1,27 (s; 9H); 1,47 (s; 9H); 2,98 (m; 1H); 3,38 (s; 1H); 3,74 (s; 3H); 3,81 (s; 3H); 3,97 (m, 1H); 4,20 (m, 1H); 6,74 (m, 2H); 6,95-7,20 (m; 18H); 7,31 (m, 2H); 7,43 (m, 2H) ppm.

### Katalyseversuche

Die Hydroformylierung wurde in einem mit Druckkonstanthaltung, Gasflussmessung, Begasungsrührer und Druckpipette ausgestatteten 200 ml-Autoklaven der Fa. Premex Reactor AG, Lengau, Schweiz, durchgeführt. Zur Minimierung eines Einflusses von Feuchtigkeit und Sauerstoff wurde das als Solvens benutzte Toluol in einem Pure Solv. MD-7 System gereinigt und unter Argon aufbewahrt. Das als Substrat eingesetzten Olefin cis/trans-2-Penten (Aldrich) wurden über Natrium am Rückfluss erhitzt und unter Argon destilliert. Im Autoklaven wurden unter Argonatmosphäre Lösungen der Katalysatorvorstufe und des Liganden, jeweils in Toluol, gemischt. Als Katalysatorvorstufe kam [(acac)Rh(COD)] (Umicore, acac = Acetylacetonat-Anion; COD = 1,5-Cyclooctadien), zum Einsatz. Der Autoklav wurde bei 12 bar für den Enddruck von 20 bar unter Rühren (1500 U/min) aufgeheizt. Nach Erreichen der Reaktionstemperatur wurde das Olefin mit einem in der Druckpipette eingestellten Überdruck in den Autoklaven gepresst. Die Reaktion wurde bei konstantem Druck (20 bar) (Nachdruckregler der Fa. Bronkhorst, NL) über 4h geführt. Der Autoklav wurde nach Ablauf der Reaktionszeit auf Zimmertemperatur abgekühlt, unter Rühren entspannt und mit Argon gespült. 1 ml der Reaktionsmischungen wurden unmittelbar nach Abschalten des Rührers entnommen, mit 10 ml Pentan verdünnt und gaschromatographisch analysiert: HP 5890 Series II plus, PONA, 50 m × 0,2 mm × 0,5 µm.

Der Versuch wurde mit den Verbindungen (**1**) und (**2**) durchgeführt.

Die Verbindung (**2**) dient hierbei als Vergleichsligand.

### Ergebnisse der Katalyseversuche

[Rh]: 100 ppm, p: 20 bar, T: 120 °C; t: 4 h; Rh:L = 1:2, Toluol

**Tabelle: Hydroformylierung von cis/trans-2-Penten**

| Ligand | Ausbeute Aldehyd [%] |
|---|---|
| (**1**)* | 78 |
| (**2**) | 74 |

| | |
|---|---|
| * erfindungsgemäße Verbindung | |

Die durchgeführten Versuche belegen, dass die gestellte Aufgabe durch eine erfindungsgemäße Verbindung gelöst wird.

## Patentansprüche

1. Verbindung gemäß Formel (**I**): 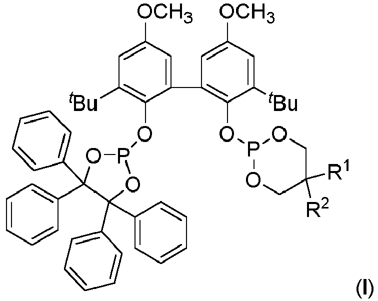 wobei R¹ für einen Phenylrest oder Naphthylrest steht, und R² für -H steht.

2. Verbindung nach Anspruch 1 wobei die Verbindung die Struktur (1) aufweist:

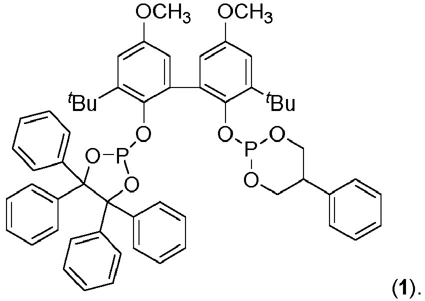

3. Verfahren umfassend die Verfahrensschritte:
a) Vorlegen eines Olefins;
b) Zugabe einer Verbindung nach einem der Ansprüche 1 oder 2;
c) Zugabe einer Rh-Verbindung;
d) Zuführen von H₂ und CO;
e) Erwärmen des Reaktionsgemisches aus a) bis d), wobei das Olefin zu einem Aldehyd umgesetzt wird.

4. Verfahren nach Anspruch 3,
wobei die Rh-Verbindung ausgewählt ist aus: Rh(acac)(CO)₂, [(acac)Rh(COD)] (Umicore, acac = Acetylacetonat-Anion; COD = 1,5-Cyclooctadien), Rh₄CO₁₂.

5. Verfahren nach einem der Ansprüche 3 oder 4,
wobei die Rh-Verbindung Rh(acac)(COD) ist.
